# EUROPEAN PATENT SPECIFICATION

(11) **EP 0 570 711 B1**
(45) Date of publication and mention of the grant of the patent: **08.07.1998**
(21) Application number: 93106506.4
(22) Date of filing: 21.04.1993
(51) Int. Cl.: A61N 1/05

(54) **Electrode device**
Elektrodenvorrichtung
Dispositif d'électrodes

(30) Priority: 21.05.1992 SE 9201599
(43) Date of publication of application: 24.11.1993
(73) Proprietor: Pacesetter AB, 171 95 Solna (SE)
(72) Inventor: Hirschberg, Jakub, S-183 44 Täby (SE)
(74) Representative: Lettström, Richard Wilhelm

(56) References cited:
- US-A- 4 763 660
- US-A- 4 938 231
- US-A- 5 063 932
- US-A- 5 105 826

## Description

The invention relates to an implantable electrode device, intended for connection to a medical device delivering electrical impulses to living tissue, comprising an electrode catheter in which an electrode lead is provided, one end of which equipped with a connection contact for the electrical lead, the other end of which connected to a flat, flexible and electrically insulative electrode carrier on which at least one electrode conductor is arranged in a predetermined pattern to form an active electrode surface, the electrode lead and the electrode conductor being electrically coupled to one another.

One such electrode device is prior art through US-A-4,817,634. The prior art electrode device comprises an insulative electrode carrier devised with a groove which follows a predetermined pattern in which an electrode conductor is placed. The electrode conductor is coiled around an insulative core and coupled to a contact clamp for electrical coupling to an electrode lead in an electrode catheter. The electrode device is designed to be sutured to the heart of a patient in order to transmit defibrillation pulses from a defibrillator to cardiac tissue.

In the implantation of the electrode device in a patient, the device is placed so it surrounds a large part of the heart, thereby more or less electrically insulating the heart from its surroundings. As a result, any external defibrillation pulse subsequently delivered to the patient could be wholly or partially ineffective, since the insulative electrode carrier would prevent electrical impulses from reaching the heart. In addition, the electrode device would also be an obstacle to any subsequent heart surgery, e.g. for removal of damaged heart tissue.

From US-A-5,105,826 there is known an implantable defibrillation electrode comprising an electrode core defining a plurality of sigmoidal curves. An electrode coil is coiled around substantially all the outside surface of the electrode core and extends along the electrode core. A proportion of the energy in an energy pulse applied to this electrode is wasted as it is applied to non-heart tissue on the side of the electrode facing away from the heart.

Another prior art electrode device is disclosed in US-A-4,567,900 and consists of an electrode devised as a resilient conductor curved in such a way as to form a ring-shaped electrode contour. Thin, flexible, conductor strips are arranged in a grid pattern in the ring-shaped contour to form an electrode surface. The ring-shaped conductor can be compressed into a narrow, elongate form which can be placed in the introducer catheter and advanced to the heart with it. The electrode is completely uninsulated and designed for loose placement between the pericardium and the epicardium. If the electrode were placed at any other location, the uninsulated electrode could damage adjacent tissue. The document does not describe anything indicating how the electrode would be prevented from shifting position before becoming embedded in cardiac tissue.

An object of the invention is to provide an electrode device of the abovementioned type which does not needlessly insulate the heart electrically from surrounding tissue, which leaves as much heart tissue as possible accessible for subsequent external measures and which does not change position after implantation.

This is achieved in accordance with the invention in that an electrode device of the abovementioned type which is designed so the electrode carrier has at least one opening in the surfaces located at the side of the predetermined pathway.

Supplying the electrode carrier with openings through which the electrode conductor does not pass results in an electrode device which is more flexible than the prior art electrode device described in US-A-4,817,634 and which can be advanced, coiled inside an introducer, into the heart in a manner similar to the method described in US-A-4,567,900 for advancing an electrode device to the heart. As a result of the openings, the electrode carrier is only located along the electrode conductor's predetermined path, so electric insulation of the heart in respect to external defibrillation is minimized, while access to heart tissue is simultaneously maximized with no reduction in the electrode's effective area. The openings also make the electrode carrier more flexible so it does not impede the heart's movements as much as an electrode carrier without openings. The carrier's ability to conform to the heart's movements also reduces the risk of any displacement of the electrode carrier's position on the heart.

An improvement of the electrode device is obtained in accordance with the invention in that the electrode catheter has a through channel, the electrode catheter is coupled to a peripheral part of the electrode carrier, a tubular element is fitted to the electrode carrier in such a way that it constitutes an extension of the electrode catheter and the tubular element is equipped with a stopper at its free end, a stylet introducible into the electrode catheter and the tubular element up to the stopper.

The stylet facilitates manipulation of the electrode device during introduction into the patient's body. With the aid of the stylet, control of placement of the electrode against tissue is improved. In addition, the contour of the electrode carrier can be changed by means of the stylet during introduction. Since the tubular element is elastic and the stopper impedes the stylet at the element's free end, the electrode carrier can be extended when the stylet is pressed against the stopper. As noted above, the openings in the electrode carrier contribute to an increase in the electrode carrier's flexibility, thereby reducing the force required to extend the electrode carrier and enabling the extended electrode carrier to become embedded in tissue without damaging the tissue which, when the stylet is withdrawn, absorbs the force required to keep the electrode carrier extended. The stylet and the stopper at the end of the tubular element can also be devised so the stylet, at a given position, is latched to the end, thereby compressing the electrode carrier when the stylet is withdrawn. In the same way as in the extension of the electrode carrier, the compressed electrode carrier can then be attached to tissue, the stylet being removed thereafter. In this manner, the electrode carrier's contour can be easily adapted to e.g. the contour of the heart in such a way that electrical pulses have the greatest possible effect.

Since the tubular element consists of a coiled further electrode conductor which is electrically coupled to the electrode lead, the active electrode surface increases without further limiting access to the heart or increasing the electrode carrier's insulative effect on the heart. In this context, it would be an advantage if the further electrode device and the electrode conductor consisted of a single conductor wire. This would result in fewer coupling points and simpler fabrication of the electrode device.

Alternately, the tubular element can consist of an extension of the electrode catheter.

An additional embodiment of the electrode device is obtained in accordance with the invention in that the electrode device at the free end of the tubular element contains a movable, fixation component for affixing the electrode device to tissue, the fixation component controllable with the stylet.

The fixation component enables the electrode device to be affixed more effectively at a desired position on the heart, making surgery less traumatic, since fixation is controlled in a remote procedure.

In this context, it would be an advantage if the stylet consisted of a double stylet, one stylet of which controlling the fixation component and the other stylet controlling the change in the electrode's contour.

In the coupling of the electrode conductor and the electrode lead, it is an advantage if the electrode conductor is coiled and if the electrode conductor and electrode lead are coiled around and/or intertwined with each other.

This would thereby reduce the number of components, since no special contact elements would be required. In the use of a plurality of electrode conductors on the electrode carrier, the pattern in which the conductors are arranged can be varied to a greater degree than when fixed contact elements are used for coupling the conductors. The electrode carrier would even retain its flexibility in the contact area.

The invention will now be described in greater detail with reference to two figures, whereby
**FIG. 1** illustrates an embodiment of the electrode device according to the invention, and
**FIG. 2** shows an enlargement of a fastening device on the electrode device.

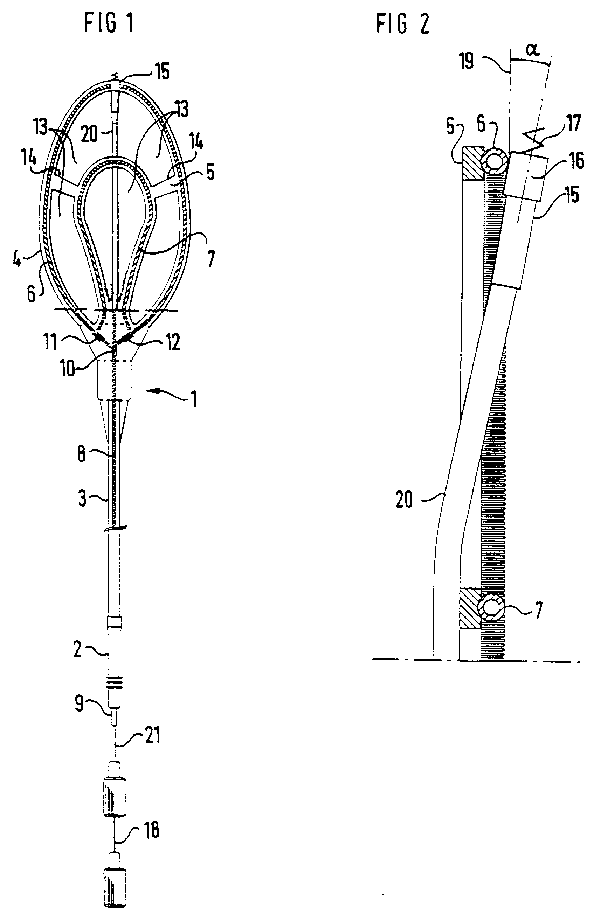

The electrode device 1 shown in FIG. 1 primarily comprises three main parts: a connection contact 2 for coupling the electrode device 1 to a stimulation pulse generator (not shown) such as a defibrillator, an electrode catheter 3 and an electrode 4 with an active electrode surface through which a stimulation pulse, generated by the defibrillator, is delivered to a heart. The electrode 4 consists of a flat, flexible, electrically insulative electrode carrier 5 , a first coiled electrode conductor 6 arranged on the periphery of the electrode carrier 5 on one side thereof, a second coiled electrode conductor 7 arranged in a simple loop inside the first electrode conductor 6 and a third coiled electrode conductor 20 attached to the electrode carrier 5 in such a way that it constitutes an extension of an electrode lead 8 running the length of the electrode catheter 3 from a terminal 9 at the connection contact 2 . The electrode lead 8 is coiled so a through channel 21 is formed in the center of the electrode catheter 3 . The first electrode conductor 6 is mechanically and electrically coupled to the electrode lead 8 in a contact area 10 by the coiling and intertwining the first electrode conductor 6 and the electrode lead 8 . In the corresponding manner, the second electrode conductor 7 is mechanically and electrically coupled to the first electrode conductor 6 at the ends of the loops 11, 12 . The electrode conductors 6, 7 and the electrode lead 8 are preferably made of a compound wire with a low-resistance core and a biocompatible sleeve. The core is appropriately made of copper, a copper compound, silver or a silver alloy with a sleeve of MP35N alloy (an alloy made of nickel, cobalt, chromium and molybdenum), titanium, a titanium alloy, platinum or a platinum alloy. The design utilizing a low-resistance core provides better and more even distribution of current over the electrode conductors 6, 7, 8 constituting the electrode's 4 active electrode surface.

The electrode carrier 5 has openings 13 between the paths of the electrode conductors 6, 7, 20 . These openings 13 serve a plurality of purposes: the electrode 4 is more flexible than an electrode without openings and can be coiled for advancing to the heart through an introducer catheter like the one described in US-A-4,567,900; the electrode carrier's insulative effect on the heart, when the electrode 4 is applied to the heart, is reduced without any reduction in the area of the active electrode so an electrical pulse, e.g. from an extracorporeal defibrillator, passes through the openings in the electrode carrier to heart tissue; and the heart is accessible through the openings for any subsequent surgery, e.g. for removal of damaged heart tissue. The different electrode loops are held together with insulation bridges 14 which also contribute to retention of the electrode 4 on one plane.

To attach the electrode 4 to the heart, the electrode device 1 has a fixation device 15 at the free end of the third electrode conductor 20 . As shown in FIG. 2, the fixation device 15 comprises a sleeve 16 and a screw 17. The screw 17 is screwed in/out of the sleeve 16 with a stylet 18 . The stylet 18 is introduced from the connection contact 2 and runs in channel 21 in the electrode catheter 3 and the third electrode conductor 20 up to the fixation device 15. The screw's 17 direction of movement follows a lead which forms a small angle α with the plane 19 of the electrode surface. As a result of the angle α , the screw 17 emerges from the electrode surface's plane 19 when screwed out so it can thereby seat itself in e.g. pericardium around the heart. Since only the pericardium is utilized for attaching the electrode to the heart, there is no damage to the epicardium. In addition, the pericardium remains generally intact, and its protection of the heart is uncompromised.

If the implantation site selected for the electrode 4 proves to be unsuitable, the screw 17 can simply be retracted back into the sleeve 16 with the stylet 18 . The electrode 4 can then be moved to some other part of the heart and seated there.

The electrode device according to the present embodiment can even be applied subcutaneously. The angle α is then preferably increased and a longer screw 17 used, depending on the sensitivity of the tissue at the electrode 4 seating site. The screw 17 can also be angled so it screws out of the back of the electrode 4 or straight out along the extension of the electrode catheter 3 . This may be preferable when less sensitive tissue is near the tissue to be stimulated. In the above example, the screw 17 is placed at the distal end of the electrode 4 but could be located anywhere along the length of the electrode 4.

Placement of the electrode 4 against the heart is additionally facilitated by the stylet 18. The electrode 4 can be moved, and its contour can even be changed, since the stylet 18 consists of a double stylet, one stylet 21 of which serving as a tool for the fixation device 15 , the other stylet altering the contour of the electrode 4 . The change in contour is accomplished when the second stylet presses against a stopper at the end of the third electrode conductor 20 in such a way that the electrode carrier 5 is stretched. The stopper can e.g. consist of the fixation device 15 . In its stretched state, the electrode carrier 5 can then be affixed to the heart, and the double stylet can thereafter be removed. In principle, one stylet 18 should suffice for controlling both the fixation device 15 and changing contour, but control over the respective function improves when a double stylet is used.

The electrode carrier 5 can also be compressed if the stylet and stopper are devised so the stylet, in a given position, cannot be withdrawn from the electrode device 1 . Retracting the stylet would compress the electrode carrier 5, enabling it to be affixed to the heart in a compressed state.

The objective of altering the contour of the electrode 4 during implantation is to increase adaptation of the contour to the individual heart and to optimize the effect of the electrical pulses, since the contour of the electrode surface governs, to some extent, the path the pulses take through heart tissue.

## Claims

1. An implantable electrode device (1), intended for connection to a medical device for delivering electrical pulses to living tissue, comprising a electrode catheter (3) in which a electrode lead (8) is provided, one end of said electrode catheter (3) being equipped with a connection contact (2) for the electrode lead (8), the other end thereof being connected to a flat, extended, flexible and electrically insulative electrode carrier (5), on one side of said electrode carrier (5) at least one electrode conductor (6) is arranged along a predetermined path to form a electrode surface only on said one side of said electrode carrier, the electrode lead (6) and the electrode conductor (8) being electrically coupled to one another, said electrode carrier (5) having at least one opening (13) in the surfaces located at the side of said predetermined path.

2. An electrode device of claim 1, wherein the electrode catheter (2) has a through channel (21), the electrode catheter (3) is coupled to a peripheral part of the electrode carrier (5), a tubular element is attached to the electrode carrier (5) in such a way that it constitutes an extension of the electrode catheter (3) and the tubular element is equipped with a stopper at its free end, a stylet (18) introducible into the electrode catheter (3) and tubular element up to the stopper.

3. An electrode device of claim 2, wherein the tubular element consists of a coiled further electrode conductor (20) which is electrically coupled to the electrode lead (8).

4. An electrode device of claim 2, wherein the further electrode conductor (20) and the electrode lead (8) consist of a single conductor wire.

5. An electrode device of claim 2, wherein the tubular element consists of an extension of the electrode catheter (3).

6. An electrode device of any of claims 2 to 5, wherein the free end of the tubular element in the electrode device contains a movable fixation component (15) for affixing the electrode device (1) to the tissue, the fixation component (15) being controllable with a stylet (18).

7. An electrode device of claim 6, wherein the stylet (18) consists of a double stylet (18, 21), one of the stylets (18) controlling the fixation component (15) and the other stylet (21) controlling the change in the contour of the electrode (4).

8. An electrode device of any of the above claims, wherein the electrode conductor (6) and the electrode lead (8) are coiled around and/or intertwined with each other to form the electrical coupling.

## Patentansprüche

1. Eine implantierbare Elektrodenvorrichtung (1), vorgesehen zum Anschließen an eine medizinische Vorrichtung zur Abgabe elektrischer Impulse an lebendes Gewebe mit einem Elektrodenkatheter (3), in dem eine Elektrodenleitung (8) vorgesehen ist, wobei ein Ende des Elektrodenkatheters (3) mit einem Anschlußkontakt (2) für die Elektrodenleitung (8) ausgestattet ist und dessen anderes Ende mit einem flachen, ausgedehnten, flexiblen und elektrische isoliertem Elektrodenträger (5) verbunden ist, wobei auf einer Seite des Elektrodenträgers (5) mindestens ein Elektrodenleiter (6) entlang eines vorbestimmten Weges angeordnet ist, um nur auf dieser einen Seite des Elektrodenträgers eine Elektrodenoberfläche zu bilden, und die Elektrodenleitung (6) und der Elektrodenleiter (8) elektrischen miteinander verbunden sind, wobei der Elektrodenträger (5) mindestens eine Öffnung (13) in der auf der Seite des vorbestimmten Weges liegenden Oberfläche hat.

2. Eine Elektrodenvorrichtung nach Anspruch 1, bei der der Elektrodenkatheter (3) einen Durchgangskanal (21) hat, der Elektrodenkatheter (3) mit einem peripheren Teil des Elektrodenträgers (5) verbunden ist, bei dem ein schlauchförmiges Element derart an dem Elektrodenträger (5) befestigt ist, daß es eine Verlängerung des Elektrodenkatheters (3) darstellt, und das schlauchförmige Element an seinem freien Ende mit einem Stöpsel versehen ist, ein in den Elektrodenkatheter (3) und das schlauchförmige Element bis hin zu dem Stöpsel einführbarer Mandrin (18).

3. Eine Elektrodenvorrichtung nach Anspruch 2, bei der das schlauchförmige Element aus einem weiteren spulenförmigen elektrischen Leiter (20) besteht, der elektrisch an die Elektrodenleitung (8) angeschlossen ist.

4. Eine Elektrodenvorrichtung nach Anspruch 2, bei der der weitere Elektrodenleiter (20) und die Elektrodenleitung (8) aus einem einzelnen Leiterdraht bestehen.

5. Eine Elektrodenvorrichtung nach Anspruch 2, bei der das schlauchförmige Element aus einer Verlängerung des Elektrodenkatheters (3) besteht.

6. Eine Elektrodenvorrichtung nach einem der Ansprüche 2 bis 5, bei der das freie Ende des schlauchförmigen Elementes in der Elektrodenvorrichtung eine bewegbare Fixierungskomponente (15) zum Befestigen der Elektrodenvorrichtung (1) an dem Gewebe enthält und die Fixierungskomponente (15) mit dem Mandrin (18) steuerbar ist.

7. Eine Elektrodenvorrichtung nach Anspruch 6 bei der der Mandrin (18) aus einem Doppelmandrin (18, 21) besteht, einer der Mandrine (18) die FiXierungskomponente (15) steuert und der andere Mandrin (21) den Wechsel in der Kontur der Elektrode (4) steuert.

8. Eine Elektrodenvorrichtung nach einem der obigen Ansprüche, bei der der Elektrodenleiter (6) und die Elektrodenleitung (8) umeinander herum und/oder ineinander gewickelt sind, um den elektrischen Anschluß zu bilden.

## Revendications

1. Dispositif (1) d'électrode implantable, destiné à être connecté à un dispositif médical pour envoyer des impulsions électriques à un tissu vivant, comportant un cathéter (3) d'électrode dans lequel un conducteur (8) d'électrode est prévu, une extrémité du cathéter (3) d'électrode étant équipée d'un contact (2) de connexion pour le conducteur (8) d'électrode, l'autre extrémité du cathéter étant connectée à un porteur (5) d'électrode, isolant électriquement, souple, étendu et plat, au moins un conducteur (6) d'électrode étant agencé, sur un côté du porteur (5) d'électrode, le long d'un trajet prédéterminé pour former une surface d'électrode uniquement sur ledit un côté du porteur d'électrode, le conducteur (6) d'électrode et le conducteur (8) d'électrode étant coupés électriquement l'un à l'autre, le porteur (5) d'électrode ayant au moins une ouverture (13) dans les surfaces située du côté du trajet prédéterminé.

2. Dispositif d'électrode suivant la revendication 1, dans lequel le cathéter (3) d'électrode a un canal (21) de traversée, le cathéter (3) d'électrode est couplé à une partie périphérique du porteur (5) d'électrode, un élément tubulaire est attaché au porteur (5) d'électrode de telle manière qu'il constitue un prolongement du cathéter (3) d'électrode et l'élément tubulaire est équipé d'une butée à son extrémité libre, un stylet (18) pouvant être introduit dans le cathéter (3) d'électrode et l'élément tubulaire jusqu'à la butée.

3. Dispositif d'électrode suivant la revendication 2, dans lequel l'élément tubulaire est constitué d'un conducteur (20) d'électrode supplémentaire enroulé qui est couplé électriquement au conducteur (8) d'électrode.

4. Dispositif d'électrode suivant la revendication 2, dans lequel le conducteur (20) d'électrode supplémentaire et le conducteur (8) d'électrode sont constitués d'un unique fil conducteur.

5. Dispositif d'électrode suivant la revendication 2, dans lequel l'élément tubulaire est constitué d'un prolongement du cathéter (3) d'électrode.

6. Dispositif d'électrode suivant l'une quelconque des revendications 2 à 5, dans lequel l'extrémité libre de l'élément tubulaire dans le dispositif d'électrode contient un composant (15) de fixation mobile pour fixer le dispositif (1) d'électrode au tissu, le composant (15) de fixation pouvant être commandé par un stylet (18).

7. Dispositif d'électrode suivant la revendication 6, dans lequel le stylet (18) est constitué d'un double stylet (18, 21), l'un des stylets (18) commandant le composant (15) de fixation et l'autre stylet (21) commandant le changement dans le contour de l'électrode (4).

8. Dispositif d'électrode suivant l'une quelconque des revendications précédentes, dans lequel le conducteur (6) d'électrode et le conducteur (8) d'électrode sont enroulés l'un autour de l'autre et/ou entrelacés l'un dans l'autre pour former le couplage électrique.
